# EUROPÄISCHE PATENTSCHRIFT

(11) **EP 1 721 160 B1**
(45) Veröffentlichungstag und Bekanntmachung des Hinweises auf die Patenterteilung: **05.05.2010**
(21) Anmeldenummer: 05707670.5
(22) Anmeldetag: 01.03.2005
(51) Int. Cl.: G01N 33/543, C12Q 1/68, C12N 11/08

(54) **VERFAHREN ZUR KOVALENTEN IMMOBILISIERUNG VON BIOMOLEKÜLEN AN POLYMEREN OBERFLÄCHEN**
METHOD FOR COVALENTLY IMMOBILISING BIOMOLECULES ON POLYMERIC SURFACES
PROCEDE POUR IMMOBILISER DE MANIERE COVALENTE DES BIOMOLECULES SUR DES SURFACES POLYMERIQUES

(30) Priorität: 01.03.2004 DE 102004010430
(43) Veröffentlichungstag der Anmeldung: 15.11.2006
(73) Patentinhaber: Rühe, Jürgen, 79356 Eichstätten (DE); Klapproth, Holger, Dr., 79108 Freiburg (DE)
(72) Erfinder: Rühe, Jürgen, 79356 Eichstätten (DE); Klapproth, Holger, Dr., 79108 Freiburg (DE)
(74) Vertreter: Huwer, Andreas
(86) Internationale Anmeldenummer: PCT/EP2005/002137
(87) Internationale Veröffentlichungsnummer: WO 2005/083435

(56) Entgegenhaltungen:
- US-A- 6 077 698
- US-A1- 2004 023 413
- US-B1- 6 346 376
- US-B1- 6 372 813
- SIGRIST H ET AL: "SURFACE IMMOBILIZATION OF BIOMOLECULES BY LIGHT" OPTICAL ENGINEERING, SOC. OF PHOTO-OPTICAL INSTRUMENTATION ENGINEERS. BELLINGHAM, US, Bd. 34, Nr. 8, 1. August 1995 (1995-08-01), Seiten 2339-2348, XP000518229 ISSN: 0091-3286
- YASUHIDE NAKAYAMA ET AL: "DESIGN AND PROPERTIES OF PHOTOCURABLE ELECTROCONDUCTIVE POLYMERS FOR USE IN BIOSENSORS" ASAIO JOURNAL, J.B.LIPPINCOTT CO.,HAGERSTOWN,MD, US, Bd. 41, Nr. 3, 1. Juli 1995 (1995-07-01), Seiten 418-421, XP000542925 ISSN: 1058-2916
- GAO H ET AL: "PHOTOLINKER-POLYMER-MEDIATED IMMOBILIZATION OF MONOCLONAL ANTIBODIES, F(AB')-2 AND F(AB') FRAGMENTS" BIOTECHNOLOGY AND APPLIED BIOCHEMISTRY, ACADEMIC PRESS, US, Bd. 20, Nr. 2, 1994, Seiten 251-263, XP009037548 ISSN: 0885-4513
- VASILISKOV A V ET AL: "FABRICATION OF MICROARRAY OF GEL-IMMOBILIZED COMPOUNDS ON A CHIP BYCOPOLYMERIZATION" BIOTECHNIQUES, EATON PUBLISHING, NATICK, US, Bd. 27, Nr. 3, September 1999 (1999-09), Seiten 592,594,596-598,600,60, XP000849476 ISSN: 0736-6205

## Beschreibung

Die Erfindung betrifft ein Verfahrens zur kovalenten Immobilisierung mindestens eines Sonden-Biomoleküls an einer Polymeroberfläche.

In den letzten Jahren haben in der Analytik Techniken immer mehr an Bedeutung gewonnen und es sind zahlreiche Festphasensysteme auf der Grundlage von selbstorganisierten Monolayern (engl. » self-assembled monolayers «, »SAMs«) aus bifunktionellen Molekülen (engl. »linker«) entwickelt worden, über die spezifisch Probenmoleküle an die Oberfläche des festen Trägers gekoppelt bzw. konjugiert werden, an der dann auch der Nachweis mit Hilfe von geeigneten Markierungen (beispielsweise radioaktiv, gefärbt fluoreszierend) erfolgt

Für diese Systeme hat sich in Analogie zu den elektronischen Mikro-Chips die Bezeichnung Sensor-Chips eingebürgert. Im Falle der Konjugation von biologischen Molekülen (sog. »Biokonjugation«) an solche Sensor-Chips, beispielsweise Oligonukleofiden oder Antikörpern, spricht man auch von Bio-Chips. Die Kopplung an die Trägeroberfläche kann direkt oder indirekt erfolgen. Ein Beispiel für eine indirekte Kopplung ist die Kopplung einer nachzuweisenden Nukleinsduresequenz durch Hybridisierung an ein immobilisiertes, komplementäres Oligonukleotid als Sonde. In diesem Fall hat die Verwendung der Sonde noch den Vorteil der natürlichen Spezifität der Wechselwirkung biologischer Makromoleküle.

Typischerweise werden zur Herstellung von Sensor-Chips Oberflächen aus Metall- bzw. Halbmetalloxiden, wie z.B. Aluminiumoxid, Quarzglas, Glas, in eine Lösung von bifunktionellen Molekülen (sog. »Linker«), die beispielsweise eine Halogensilan- (z.B. Chlorsilan-) oder Alkoxysilangruppe zur Kopplung an die Trägeroberfläche aufweisen, getaucht, so dass sich ein selbstorganisierter Monolayer (SAM) bildet. Dieser weist in diesem Fall eine Dicke von wenigen Ångström aus. Die Kopplung der Linker an die Proben- oder Sondenmoleküle erfolgt über eine geeignete weitere funkfionelle Gruppe, beispielsweise eine Amino- oder Epoxygruppe (EP 1176 422 A1). Geeignete bifunktionelle Linker für die Kopplung einer Vielzahl von Proben- oder Sonden-Molekülen, insbesondere auch biologischen Ursprungs, an eine Vielzahl von Trägeroberflächen sind dem Fachmann gut bekannt, vgl. beispielsweise »Bioconjugate Techniques« von G. T. Hermanson, Academic Press 1996.

Ein Nachteil dieser reaktiven (und dadurch empfindlichen) Oberflächen, z.B. Oberflächen mit Epoxy-, Aldehyd- oder Aminofunktionen, ist deren oft nur begrenzte Haltbarkeit (wenige Wochen), so dass sie unter Luftabschluss und / oder im Dunklen gelagert werden müssen. Zudem sind die resultierenden Bindungen zu den Biomolekülen oder der Oberfläche nicht langzeitstabil. Alle diese Bindungen unterliegen Effekten, wie der Hydrolyse, wodurch die Verwendbarkeit und das Anwendungsspektrum gravierend eingeschränkt werden. Insbesondere erlauben es die bestehenden Methoden nicht, auf hydrophile Oberflächen, bzw. hydrophil beschichtete Oberflächen zu drucken, ohne dass die Gefahr besteht, dass die Tropfen zerlaufen und dadurch das Druckergebnis zerstört wird.

Die Immobilisierung von beispielsweise Nukleinsäuren auf nicht reaktiven Polymer- bzw. Kunststoff /Plastikoberfiächen (z. B. als Sonden zur Herstellung von Sensor-/Bio-Chips) mit herkömmlichen Methoden ist aber kompliziert und erfordert sehr viel Aufwand.

Aus Gao H. et al.: "Photolinker-polymer-mediated immobilization of monoclonal antibodies, F(ab')2 and F(ab') fragments", Biotechnol. Appl. Biochem., 20 (2), 1994, Seite 251-263 ist ein Verfahren zur kovalenten Immobilisierung von Sonden-Biomolekülen an einer Polymeroberfläche, nämlich einer Polystyren-Oberfläche, bekannt. Die Polymeroberfläche wird mit einem photoreaktive Aryldiazirin-Gruppen aufweisenden Polymer beschichtet und danach getrocknet. In einem Lösungsmittel werden Sonden-Biomoleküle, nämlich Antikörper gelöst. Die Lösung wird auf die zuvor beschichtete Polymeroberfläche aufgebracht und danach entfernt. Die so erhaltene Oberfläche wird mit Licht der Wellenlänge 350 nm bestrahlt Dabei entstehen Carbene, die an die Biomoleküle und die Polymeroberfidche binden. Das Verfahren ist relativ aufwändig, weil die Polymeroberfläche mehrfach beschichtet wird. Ungünstig ist außerdem, dass das Verfahren eine lange Bestrahlungsdauer erfordert.

Aus Sigrist, H. et al.: Surface immoblization of biomolecules by light", Optical Engeneering, Bd. 34, Nr. 8, 1. August 1995, Seiten 2339-2348 ist ferner ein Verfahren bekannt, bei dem mindestens ein Sonden-Biomolekül mit mit wenigstens einem Polymer, das mindestens zwei photoreaktive Gruppen pro Molekül aufweist, gelöst wird. Die Mischung wird auf eine anorganische TiO₂ oder SiO₂-Oberfläche aufgebracht und derart mit Licht bestrahlt, dass eine Copolymerisafion auftritt, bei der aus unterschiedlichen kurzkettigen Polymeren ein langkettiges Copolymer gebildet wird. Dabei werden die Biomoleküle kovalent an das Copolymer und das Copolymer kovalent an die Oberfläche gebunden. Die Autoren empfehlen, für die Bestrahlung eine Wellenlänge von etwa 350 nm zu verwenden, damit die Sonden-Biomoleküle nicht geschädigt werden.

Aufgabe der Erfindung ist daher die Bereitstellung eines einfachen und schnell durchzuführenden Verfahrens zur kovalenten Immobilisierung von Sonden-Biomolekülen an organischen Oberflächen.

Erfindungsgemäß wird diese Aufgabe mit den Merkmalen des Anspruchs 1 gelöst

Ein Vorteil der Erfindung liegt in der Möglichkeit, auf unreaktive Oberflächen (z.B. Substrate aus handelsüblichen Kunststoffen) ein viskoses Medium, zu drucken, das sehr einfach zu immobilisieren ist, nämlich durch Bestrahlung mit Licht einer geeigneten Wellenlänge. Gleichzeitig wird durch diesen Vorgang die Menge an Analyt, die gekoppelt werden kann, wesentlich erhöht, da eine pseudo-dreidimensionale Matrix aufgebaut wird. Klassische Probleme dreidimensionaler Matrizes, wie z.B. Verlaufeffekte des Mediums beim Drucken auf Polymergele, werden auf diese Weise zusätzlich gelöst. Zudem muss nicht auf reaktive (und dadurch empfindliche) Oberflächen gedruckt werden. Reaktive Oberflächen sind z.B. Oberflächen mit Epoxy, Aldehyd- oder Aminofunktionen. Reaktive Oberflächen weisen oft nur eine begrenzte Haltbarkeit (wenige Wochen) auf und müssen unter Lufrabschluss gelagert werden. Keine reaktive Oberfläche bedeutet, dass Träger aus z.B. Polystyrol oder Polymethylmethacrylat (PMMA) verwendet werden können, die jahrelang stabil sind. Ein weiter Vorteil ist, dass z.B. die Polymeroberflächen nicht durch vorgeschaltete Prozessschritte wie z.B. Plasmaprozesse hydrophilisiert werden müssen, da die Zugänglichkeit der Oberfläche zum Beispiel bei der oben definierten alternativen Ausführungsform des erfindungsgemäßen Verfahrens durch das gekoppelte (quellbare, benetzbare) Copolymer hergestellt wird. Abgesehen davon sind außerdem die Oberflächeneigenschaften des Substrats (z.B. der Sensoroberfläche) in einfacher Weise sehr genau zu kontrollieren. Ein Beispiel für eine wichtige Obernächeneigenschaff, die mit Hilfe des hier beschriebenen Verfahrens einfach kontrolliert werden kann, ist die Benetzbarkeit. Ein weiterer Vorteil ist die vereinfachte Analytik, da im Prinzip nur das Volumen des aufgebrachten Tropfens bestimmt werden muss und sich daraus die Anzahl der immobilisierten Sonden unmittelbar ergibt. Dies ist bei den Verfahren des Standes der Technik zur Bindung von beispielsweise DNA an SAMs kein triviales Unterfangen.

Mit dem erfindungsgemäßen Verfahren kann eine hohe Bindekapazität auf Trägersubstraten erreicht werden. Bisherige Ansätze wie z.B. EP 1 144 677 A2 konnten dieses Problem nicht zufrieden steigend lösen. Das erfindungsgemäße Verfahren ermöglicht außerdem die Bereitstellung einer stabilen Kopplungschemie und einer verbesserten Bedruckbarkeit von hydrophilen und hydrophoben Oberflächen.

Die Erfindung betrifft ferner eine Polymeroberfläche mit kovalent, vorzugsweise unter Musterbildung (z.B. durch Aufdrucken), darauf immobilisierten Sonden-Biomolekülen, die nach einem oben definierten Verwahren erhältlich ist.

Die Erfindung ermöglicht die Verwendung einer organischen Polymeroberfläche, mit unter Musterbildung darauf immobilisierten Sonden-Biomolekülen als Sensor-Chip. Die erfindungsgemäße Oberfläche mit den darauf immobilisierten Sonden-Biomolekülen kann außerdem in einem medizinischen oder diagnostischen Instrument enthalten sein.

Vorteilhafte und/oder bevorzugte Ausführungsformen der Erfindung sind Gegenstand der Unteransprüche.

Für das erfindungsgemäße Verfahren eignen sich Polymeroberflächen aus Cycloolefincopolymeren (COCs), Polysty-rol, Polyethylen, Polypropylen oder Polymethylmethacrylat (PMMA, Plexiglas). Ein geeignetes COC ist zum Beispiel das von Ticona unter dem Handelsnamen »Topas« vertriebene.

In dem erfindungsgemäßen Verfahren kann das Sonden-Biomolekül beispielsweise ein Partner eines spezifisch wechselwirkenden Systems von komplementären Bindungspartnern (Rezeptor/Ligand) sein.

Rezeptoren sind beispielsweise, aber nicht ausschließlich: Nukleinsäuren und deren Derivate (RNA, DNA, LNA, PNA), Proteine, Peptide, Polypetide und deren Derivate (Glycosamine, Antikörper, Enzyme) aber auch Fettsäuren wie z.B. Arachidonsäure und andere Verbindungen, sofern diese spezifische Wechselwirkungen mit mindestens einem zweiten Molekül eingehen können. Weiterhin größere und zusammengesetzte Strukturen wie z.B. Lioposomen, Membranen und Membranfragmente, Zellen, Zelllysate, Zellfragmente, Sporen und Mikroorganismen.

Liganden sind beispielsweise, aber nicht ausschließlich: Nukleinsäuren und deren Derivate (RNA, DNA, LNA, PNA), Proteine, Peptide, Polypetide und deren Derivate (Glycosamine, Antikörper, Enzyme) aber auch Fettsäuren wie z.B. Arachidonsäure und andere Verbindungen, sofern diese spezifische Wechselwirkungen mit mindestens einem zweiten Molekül eingehen können. Weiterhin größere und zusammengesetzte Strukturen wie z.B. Lioposomen, Membranen und Membranfragmente, Zellen, Zelllysate, Zellfragmente, Sporen und Mikroorganismen.

Ein spezifisch wechselwirkendes System von komplementären Bindungspartnern kann beispielsweise auf der Wechselwirkung einer Nukleinsäure mit einer komplementären Nukleinsäure, der Wechselwirkung einer Peptidnukleinsäure (PNA) mit einer Nukleinsäure, der Enzym/Substrat; Rezeptor/Ligand-, Lectin/Zucker; Antikörper/Antigen-, Avidin/Biotin- oder Streptavidin/Biotin-Wechseiwirkung beruhen.

Natürlich kann die Nukleinsäure eine DNA oder RNA sein, zB. ein Oligonukleotid oder ein Aptamer oder auch eine sog. »LNA« wie unter www.proligo.com angeboten oder auch eine einpolymerisierbare DNA wie unter dem Handelsnamen »Acrydite« unter www.mosaic-technologies.com angeboten. Auch Peptidnukleinsäuren (PNAs) kommen In Frage.

Bei dem Antikörper kann es sich beispielsweise um einen polyklonalen, monoklonalen, chimären oder »Single-chain«-Antikörper oder ein funktionelles Fragment oder Derivat (mit »funktionell« ist gemeint, dass das Fragment/Derivat ein Antigen binden kann, ohne dass notwendigerweise eine Immunogenität damit verbunden ist) eines derartigen Antikörpers handeln.

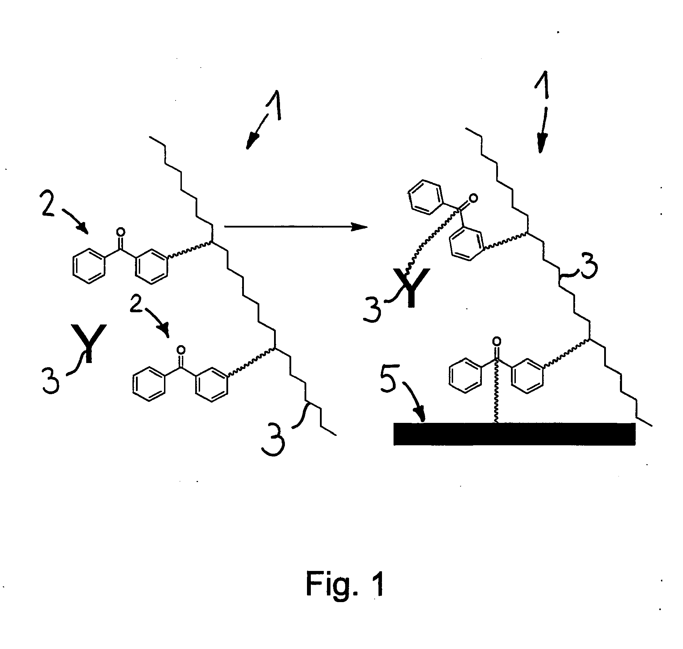

Im Folgenden wird die Erfindung ohne Beschränkung unter Bezugnahme auf konkrete Ausführungsformen und Beispiele anhand von Nukleinsäuren als Sonden-Biomolekülen detaillierter erläutert. Es zeigt
Fig. 1 das Vernetzen von Biomolekülen und einem Copolymer in schematischer Darstellung.

### Herstellung des Copolymers:

Ein in Fig. 1 mit 1 bezeichnetes Copolymer kann aus einem eine UV-reaktive Gruppe 2 aufweisenden Monomer 3, einem reaktiven hydrophilen Monomer gebildet werden. Z.B. 4-Methacryloyloxybenzophenon, Dimethylacrylamid und Methacrylsäure.

Beispielsweise kann durch Copolymersisation von Dimethylacrylamid und 4-Methacryloyloxybenzophenon in einem 100:1 (mol/mol) Gemisch durch Zugabe von 1 % AIBN (Azobisisobutyronitril) in eine Lösung der Monomeren in einem geeigneten Lösungsmittel (z.B. 10% (v/v) Monomere in Chloroform) hergestellt werden. Das entstandene Copolymer kann durch Fällen mit Diethylether abgetrennt werden.

Das Copolymer 1 kann zum Beispiel in einem geeigneten Lösungsmittel gequollen und zum Beispiel mit 5' Oligothymin modifizierter Nukleinsäure wie DNA gemischt werden.

Das so erhaltene, in Fig. 1 links gezeigte Gemisch aus Biomolekül 4 und Copolymer 1 kann nun vermessen werden (um den DNA-Gehalt zu bestimmen) und auf fast beliebige organische Polymeroberflächen 5 als Substrat durch Drucken aufgebracht werden (Fig. 1 rechts). Die Immobilisierung des Polymers und die Vernetzung mit dem Biomolekül 4 erfolgt über UV-Bestrahlung bei einer Wellenlänge von 260 nm.

Dieses Polymer kann über ein dem Fachmann bekanntes Verfahren auf ein PMMA-Substrat gedruckt werden. Die Immobilisierung der modifizierten Polymere erfolgt hier zum einen über eine photoinduzierte Kupplungsreaktion zwischen den im Polymer enthaltenen Benzophenon-Gruppen und dem Substrat, ausgelöst durch UV-Bestrahlung bei 260 nm und einer photoinduzierten Kupplungsreaktion zwischen dem Oligothymin und dem Polymer und/oder der photoinduzierten Kupplungsreaktion zwischen dem Benzophenon und der Nukleinsäure.

## Patentansprüche

1. Verfahren zur kovalenten Immobilisierung mindestens eines Sonden-Biomoleküls an einer Polymeroberfläche, bei dem
(a) das mindestens eine Sonden-Biomolekül mit wenigstens einem Polymer und/oder Copolymer, das mindestens zwei photoreaktive Benzophenon-Gruppen pro Molekül aufweist, gelöst wird,
(b) die Mischung aus (a) auf die Polymeroberfläche aufgebracht und
(c) derart mit Licht einer Wellenlänge von 260 nm bestrahlt wird,
- dass das mindestens eine Sonden-Biomolekül kovalent an das Polymer und/oder Copolymer gebunden wird, und
- dass das Polymer und/oder Copolymer kovalent an die Polymeroberfläche gebunden und quervernetzt wird.

2. Verfahren nach Anspruch 1, wobei das Polymer ein quellbares Polymer ist, in dem pro Polymerkette mindestens zwei identische oder unterschiedliche photovemetzbare Gruppen vorhanden sind und/oder das Copolymer ein quellbares Copolymer ist, in dem pro Copolymerkette mindestens zwei identische oder unterschiedliche photovemetzbare Gruppen vorhanden sind.

3. Verfahren nach Anspruch 1 oder 2, wobei das Polymer und/oder Copolymer durch Drucken auf die Oberfläche aufgebracht und danach quervernetzt wird.

4. Verfahren nach einem der Ansprüche 1 bis 3, wobei in dem in Anspruch 1 definierten Schritt (b) die Aufbringung durch Aufdrucken unter Musterbildung erfolgt.

5. Verfahren nach einem der Ansprüche 1 bis 4, wobei die Polymeroberfläche aus Cycloolefincopolymeren, Polystyrol, Polyethylen, Polypropylen oder Polymethylmethacrylat besteht.

6. Verfahren nach einem der Ansprüche 1 bis 5, wobei als Sonden-Biomolekül ein Partner eines spezifisch wechselwirkenden Systems von komplementären Bindungspartnern (Rezeptor/Ligand) verwendet wird.

7. Verfahren nach Anspruch 6, wobei das spezifisch wechselwirkende System von komplementären Bindungspartnern auf der Wechselwirkung einer Nukleinsäure mit einer komplementären Nukleinsäure, der Wechselwirkung einer Peptidnukleinsäure mit einer Nukleinsäure, der Enzym/Substrat, Rezeptor/Effektor-, Lectin/Zucker-, Antikörper/Antigen-, Avidin/Biotin- oder -Streptavidin/Biofin-Wechselwirkung beruht.

8. Verfahren nach Anspruch 7, wobei die Nukleinsäure eine DNA oder RNA oder ein Analogon davon ist.

9. Verfahren nach Anspruch 8, wobei die DNA oder RNA ein Oligonukleotid ist.

10. Verfahren nach Anspruch 7, wobei der Antikörper ein polyklonaler, monoklonaler, chimärer oder »Single-chain«-Antikörper oder ein funktionelles Fragment oder Derivat eines derartigen Antikörpers ist.

## Claims

1. Method for covalent immobilization of at least one probe-biomolecule on a polymer surface, the method comprising
(a) dissolving the at least one probe-biomolecule with at least one polymer and/or copolymer having at least two photoreactive benzophenone groups per molecule,
(b) applying the mixture from (a) to the polymer surface, and
(c) irradiating with light of a wavelength of 260 nm such that
- the at least one probe-biomolecule becomes covalently bonded to the polymer and/or copolymer, and
- the polymer and/or copolymer becomes covalently bonded to the polymer surface and cross-links.

2. Method according to Claim 1, wherein the polymer is a swellable polymer, in which there are at least two identical or different photocross-linkable groups per polymer chain and/or the copolymer is a swellable copolymer, in which there are at least two identical or different photocross-linkable groups per copolymer chain.

3. Method according to Claim 1 or 2, wherein the polymer and/or copolymer is/are applied to the surface by printing and then cross-linked afterwards.

4. Method according to any of Claims 1 to 3, wherein the application in step (b) defined in Claim 1 is effected with the formation of a pattern by printing.

5. Method according to any of Claims 1 to 4, wherein the polymer surface consists of cycloolefin copolymers, polystyrene, polyethylene, polypropylene or polymethylmethacrylate.

6. Method according to any of Claims 1 to 5, wherein a partner of a specifically interacting system of complementary bonding partners (receptor/ligand) is used as a probe-biomolecule.

7. Method according to Claim 6, wherein the specifically interacting system of complementary bonding partners is based on the interaction of a nucleic acid with a complementary nucleic acid, the interaction of a peptide nucleic acid with a nucleic acid, or the enzyme/substrate, receptor/effector, lectin/sugar, antibody/antigen, avidin/biotin or streptavidin/biotin interaction.

8. Method according to Claim 7, wherein the nucleic acid is a DNA or an RNA or an analog thereof.

9. Method according to Claim 8, wherein the DNA or RNA is an oligonucleotide.

10. Method according to Claim 7, wherein the antibody is a polyclonal, monoclonal, chimeric, or "single chain" antibody or a functional fragment or a derivative of such an antibody.

## Revendications

1. Procédé pour l'immobilisation par covalence d'au moins une biomolécule de type sonde sur une surface polymère, dans lequel
(a) ladite au moins une biomolécule de type sonde est dissoute avec au moins un polymère et/ou copolymère qui présente au moins deux groupes benzophénone photoréactifs par molécule,
(b) le mélange de (a) est appliqué sur la surface polymère et
(c) est irradié par de la lumière d'une longueur d'onde de 260 nm de manière telle
- que ladite au moins une biomolécule de type sonde est liée par covalence au polymère et/ou copolymère, et
- que le polymère et/ou le copolymère est lié par covalence à la surface du polymère et est réticulé.

2. Procédé selon la revendication 1, où le polymère est un polymère gonflable, dans lequel sont présents, par chaîne de polymère, au moins deux groupes photoréticulables identiques ou différents et/ou le copolymère est un copolymère gonflable dans lequel sont présents, par chaîne de copolymère, au moins deux groupes photoréticulables identiques ou différents.

3. Procédé selon la revendication 1 ou 2, où le polymère et/ou le copolymère est appliqué par impression sur la surface, puis est réticulé.

4. Procédé selon l'une quelconque des revendications 1 à 3, où, dans l'étape (b) définie dans la revendication 1, l'application est réalisée par impression avec formation d'un motif.

5. Procédé selon l'une quelconque des revendications 1 à 4, où la surface polymère est constituée par des copolymères de cyclooléfine, du polystyrène, du polyéthylène, du polypropylène ou du poly(méthacrylate de méthyle).

6. Procédé selon l'une quelconque des revendications 1 à 5, où on utilise, comme biomolécule de type sonde, un partenaire d'un système à interaction spécifique de partenaires de liaison complémentaires (récepteur/ligand).

7. Procédé selon la revendication 6, où le système à interaction spécifique de partenaires de liaison complémentaires repose sur l'interaction d'un acide nucléique avec un acide nucléique complémentaire, sur l'interaction d'un acide nucléique peptidique avec un acide nucléique, sur l'interaction enzyme/substrat, récepteur/effecteur, lectine/sucre, anticorps/antigène, avidine/biotine ou streptavidine/biotine.

8. Procédé selon la revendication 7, où l'acide nucléique est un ADN ou un ARN ou un analogue de ceux-ci.

9. Procédé selon la revendication 8, où l'ADN ou l'ARN est un oligonucléotide.

10. Procédé selon la revendication 7, où l'anticorps est un anticorps polyclonal, monoclonal, chimère ou à simple chaîne ou un fragment ou dérivé fonctionnel d'un tel anticorps.
